# EUROPEAN PATENT SPECIFICATION

(11) **EP 0 565 674 B1**
(45) Date of publication and mention of the grant of the patent: **07.01.1999**
(21) Application number: 92922231.3
(22) Date of filing: 20.10.1992
(51) Int. Cl.: A61K 31/56, A61K 31/57

(54) **METHOD FOR MEMORY ENHANCEMENT AND QUALITY OF LIFE IMPROVEMENT**
VERFAHREN ZUR VERBESSERUNG DES GEDÄCHTNISSES UND ZUR STEIGERUNG DER LEBENSQUALITET
PROCEDE DE STIMULATION DE LA MEMOIRE ET D'AMELIORATION DE LA QUALITE DE VIE

(30) Priority: 22.10.1991 US 780476
(43) Date of publication of application: 20.10.1993
(73) Proprietor: CITY OF HOPE, Duarte California 91010-0269 (US)
(72) Inventor: ROBERTS, Eugene, Monrovia, CA 91016 (US)
(74) Representative: Hall, Marina
(86) International application number: US9208935
(87) International publication number: WO9307877

(56) References cited:
- US-A- 4 812 447
- US-A- 5 017 470
- PROCEEDINGS OF THE NATIONAL ACADEMY OF SCIENCES vol. 89, no. 5 , 1 March 1992 pages 1567 - 1571 FLOOD, J. F. ET AL 'MEMORY-ENHANCING EFFECTS IN MALE MICE OF PREGNENOLONE AND STEROIDS METABOLICALLY DERIVED FROM IT' *
- BRAIN RESEARCH vol. 406, no. 1/2 , 17 March 1987 pages 357 - 362 ROBERTS, E. ET AL 'EFFECTS OF DEHYDROEPIANDROSTERONE SULFATE ON BRAIN TISSUE IN CULTURE AND ON MEMORY IN MICE' *
- BRAIN RESEARCH vol. 448, no. 1 , 1988 pages 178 - 181 FLOOD, J.F. ET AL 'DEHYDROEPIANDROSTERONE SULFATE IMPROVES MEMORY IN AGING MICE' *

## Description

### Field of the Invention

This application is a continuation of United States application Serial No. 07/780,476 filed 22 October 1991.

This invention relates to memory enhancement and to improvement in the quality of life for aging human individuals. More particularly, the invention relates to the enhancement of memory and quality of life by the administration of pregnenolone, pregnenolone sulfate and certain structurally similar organic compounds.

### Background of the Invention

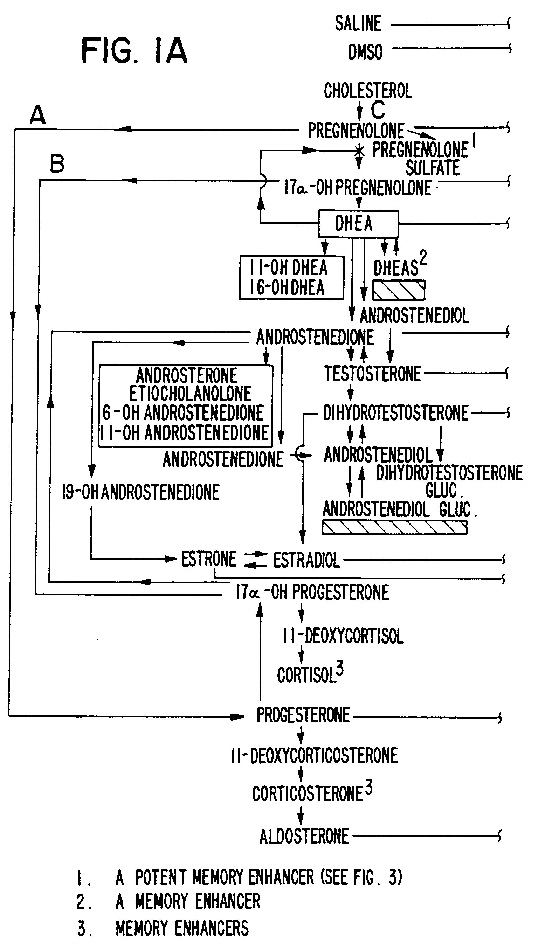

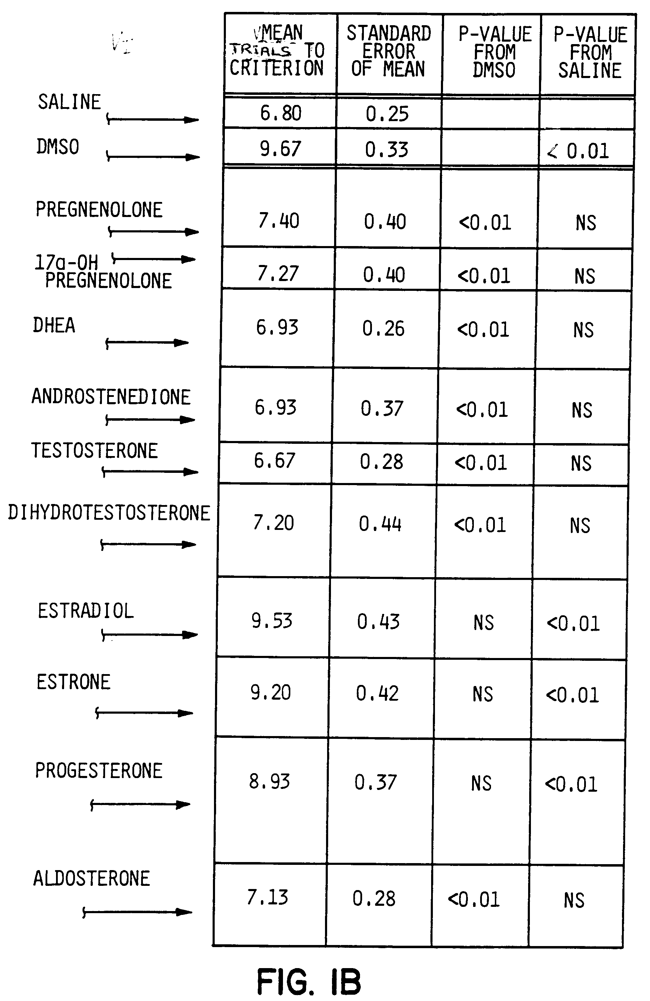

Figures 1A and 1B, considered together, are a partial outline of the steroid metabolic scheme as it may occur in the human organism as a whole. See Parker, L.N., Adrenal Androgens in Clinical Medicine, Academic Press, New York, p. 615 (1989). If the entire organism were homogenized and the homogenate appropriately extracted, one could expect to find the substances shown in Figures 1A and 1B to demonstrate the activities of enzymes that catalyze the indicated interconversions. However, it is highly unlikely that any single tissue contains all of the substances and/or enzymes, and among those tissues in which they do exist, marked differences in levels would be found. Perhaps all cells in the body require for regulation of their functions some or all of these steroids, whose various activities range from modulation of membrane excitability to regulation of genomic transcription. The blood furnishes the common reservoir from which these substances can be withdrawn by tissues to meet their needs and to which they can contribute excesses that are produced. Serum levels of these substances are a resultant of synthesis, secretion and transport in different cell types, which processes are controlled by varieties of feedback and feed-forward signals.

Pregnenolone can go directly to progesterone and thence to aldosterone (route A, Figures 1A and 1B) or to 17α-OH-pregnenolone, which is a precursor for cortisol formation (route B, Figures 1A and 1B) and for sex-related steroids (route C, Figures 1A and 1B). Route A can contribute to route B and route B to route C, as shown. DHEA, dehyroepiandrosterone, the first product in route C, may inhibit the flow through routes B and C by inhibiting conversion of pregnenolone to 17α-OH pregnenolone.

The biosynthesis of steroid hormones begins with cholesterol, from which the sex steroids, glucorticoids, and mineralocorticoids all eventually derive (see Figures 1A and 1B). Pregnenolone, a key cholesterol metabolite, is the major precursor for the steroid hormones. Its formation is rate-limiting and is regulated by pituitary hormones, such as luteinizing hormone (LH) and follicle-stimulating hormone (FSH) in ovaries and testes and ACTH and possibly a non-ACTH pituitary peptide in adrenals. Determinations were made in several regions of human brains and in cranial nerves of the contents of pregnenolone, pregnenolone sulfate, DHEA, DHEAS, dehydroepiandrosterone sulfate, androstenedione, testosterone, dihydrotestosterone, estrone and estradiol. See Lanthier, A., et al., J.Steroid Biochem. 25:445-449 (1986). The highest values in brain were found by Lanthier, supra for pregnenolone, DHEA and DHEAS. See Table 1.

**TABLE 1**

| Pregnenolone, DHEA and DHEAS in Several Regions of Human Brain (ng/100 gm)^{a/} Comparison with Blood Levels (ng/100 ml)^{b/} | | | |
|---|---|---|---|
| Brain Region | Pregnenolone | DHEA | DHEAS |
| Frontal cortex | 4074 | 1962 (0.48)^{c/} | 483 (0.12) |
| Hippocampus | 3749 | 1680 (0.45) | 459 (0.12) |
| Parietal cortex | 3620 | 1930 (0.53) | 264 (0.07) |
| Temporal cortex | 3485 | 1568 (0.45) | 305 (0.09) |
| Amygdala | 3201 | 1578 (0.49) | 483 (0.15) |
| Hypothalamus | 2786 | 1235 (0.44) | 364 (0.13) |
| Blood | 111 | 455 (4.10) | 270,000 (2,432) |

| | | | |
|---|---|---|---|
| a/ Means of 4 to 5 brains from individuals of both sexes. | | | |
| b/ Means from 50 healthy individuals of both sexes, 20 to 50 years of age. | | | |
| c/ Numbers in parentheses are ratios to pregnenolone. | | | |

The orders of contents in brain and blood are reversed: brain, pregnenolone > DHEA > DHEAS; blood, DHEAS > > DHEA > pregnenolone. Ibid.

There appear to be separate regulatory mechanisms for the above three substances in brain and blood, their metabolism being largely indigenous in brain. See Le Goascogne, C., et al., Science 237:1212-1215 (1987). Cytochrome P-450scc, the enzyme involved in pregnenolone formation, is largely localized to white matter and probably present in the glial compartment (oligodendrocytes). Ibid. In view of the large concentration gradient between brain and blood in addition to the adrenal gland, the brain may be a source of blood pregnenolone, from which synthesis of other steriods may take place in the several tissues of the body.

In this context, it is noteworthy that applicant has observed greatly reduced levels of pregnenolone in blood of individuals with Alzheimer's disease (AD). Six patients definitively diagnosed to have AD, aged 88, 75, 72, 73, 75 and 67 years, respectively, had values of pregnenolone of 68, 64, 0, 25, 33 and 35 ng/DL, respectively. These values are far below the normal control values obtained from 50 healthy individuals, 20-50 years of age: mean and range, 111 (46-225) ng/DL. All of the AD values were below the mean value of the normal range, and four of the six were below the lowest value of the range.

Pregnenolone is formed from cholesterol in mitochondria of those tissues that produce steroid hormones. Pregnenolone sulfate is formed from pregnenolone by a sulfotransferase and can be returned to pregnenolone by a sulfatase. The rate of steroid synthesis in the zona fasciculata of the adrenal cortex is controlled by the delivery of cholesterol from cytoplasmic inclusion droplets to the inner mitochondrial membrane, where steroidogenesis begins by production of pregnenolone from cholesterol by action of cytochrome P-450scc (side-chain cleavage enzyme), a reaction also found in the brain. Recently, an adrenal protein, des-(gly-Ile)-endozepine, has been isolated and characterized, the synthesis of which is induced by ACTH and which stimulates delivery of cholesterol to the inner mitochondrial membrane and possibly assists in the mobilization of inner membrane cholesterol. See Besman, M.J., et al., Pro.Natl.Acad.Sci.USA 86:4897-4901 (1989). Similar processes may take place in the brain.

DHEA and DHEAS fit into the steroid metabolic scheme in the following fashion. In the human, pregnenolone goes via 17α-OH-pregnenolone to DHEA, from which is formed the androgen, testosterone, in the testes, ovaries, adrenal cortex, and placenta. Testosterone is a precursor of the equally potent androgen, dihydrotestosterone, in the prostate, skin, hair follicles, and brain. Testosterone also is the precursor for estrogen formation by aromatization. In the female, the latter takes place largely in the ovaries, but estrogens also are formed from androgenic precursors in both males and females in other tissues, including muscle, adipose tissues, liver and brain. The rate of estrogen formation from circulating androgens is increased by hyperthyroidism and certain forms of liver disease and obesity. It is decreased in some other pathological states. See Parker, supra.

DHEAS is the most abundant steroid in the blood, its levels being far higher than those of DHEA, from which it can be formed by sulfotransferase and to which it can be converted by a sulfatase. Serum levels of DHEA and DHEAS normally largely are determined by synthesis and secretion from the adrenal cortex, but also to some extent from other tissues, such as the brain.

Low serum values of DHEA and DHEAS found at birth persist through the sixth year of life and then rise abruptly at the seventh year. See de Peretti, E., et al., J.Clin. Endocrinol. Metab. 47:572-577 (1978). Increases continue until approximately the sixteenth year in both sexes, but only in males do they increase thereafter; maximal levels being attained between 20 and 24 years of age. From puberty on, the blood levels of men are significantly higher than those of women at all ages from 20-69 years, probably reflecting a testicular contribution to the serum pool. See Orentreich, N., et al., J.Clin. Endocrinol. Metab. 59:551-555 (1984). Of particular significance in the context of this invention is the observation that progressive declines in serum concentrations of these steroids take place in both sexes at all subsequent ages; values at 70 years of age being approximately only 20% of those found at the peak. This also appears to be the case for pregnenolone and pregnenolone sulfate.

Probably all of the above hormones exert separate effects on tissues by binding to specific receptors, whose activation leads, often through effects on cyclic nucleotide mechanisms, to the release of rate-limiting reactions at consequent steps of relevant cascades. Even if normal levels of hypothalamic and pituitary hormones were to be maintained with aging, the receptor sensitivity or metabolic aspects of the ensuing reaction cascade of the responding tissues might be decreased. For example, a study of aging humans showed there to be no impairment of the ability of the adrenals to respond to acute ACTH stimulation with production and secretion of cortisol, but there was a significant decrease in stimulatability of secretion of DHEA and DHEAS. See Parker, supra. Another study showed that in older individuals (72-102 years old, average 85 years), basal levels of cortisol and aldosterone and the ACTH-stimulatability of their formation and release were essentially the same as in younger individuals (35-62 years old, average 51 years); but the basal levels of DHEA and DHEAS were significantly higher in the younger group than in the older one and no stimulation at all by ACTH of increase in serum levels of these substances was observed in the older group. See Parker, supra. Thus, not only do the serum levels of DHEA and DHEAS fall with age similarly in both sexes, but also the stimulatability of the release of these substances by ACTH from the adrenal cortex is markedly reduced in the aging human organism.

### Summary of the Invention

To date, members of the individual classes of hormones have been administered to correct one or another defect associated with specific biological effects of the particular hormone. In no instance known to applicant has pregnenolone or its sulfate been given with a view to reestablishing optimal balance between members of the different classes of these hormones.

This invention provides a method for correcting the imbalance between the steroid hormones consequent from aging by the administration of pregnenolone or pregnenolone sulfate, thus making available sufficient pregnenolone to afford the various tissues sufficient precusor for synthesis of the properly adaptive mix of the steroid hormones necessary for maintenance of functional and metabolic integrity. In this way, memory may be enhanced and the quality of life for aging human individuals may be improved.

In particular, this invention entails the discovery that pregnenolone and pregnenolone sulfate appear to enhance memory significantly and to restore vigor and sexual function associated with aging in men and women.

### Detailed Description of the Invention

Administration of pregnenolone and pregnenolone sulfate orally, subcutaneously, intravenously, transcutaneously, intrathecally, or intracisternally leads to the controlled production of a full and well-balanced spectrum of steroidal hormones, the proper balance of which is required for optimal protection against damaging influences of aging, infections, and autoimmunity and which will facilitate regeneration of tissues when injuries occur, for whatever reasons. Thus, administration of these substances appears to exert remarkable recyberneticizing effects on the nervous system and other bodily systems and on their relations to each other.

### Exemplification of the Invention

### Materials and Methods

Test Animals. After 1 week in the laboratory, CD-1 male mice obtained from Charles River Breeding Laboratories were caged individually 24-48 hr prior to training and remained singly housed until retention was tested 1 week later. Animal rooms were on a 12-hr light/dark cycle with lights going on at the hour of 0600. Median body weight was 35 g, with a range of 33-38 g. Mice were assigned randomly to groups of 15 in the experiments reported in Figure 1 and groups of 10 for the dose-response curves (Figures 2 and 3) and were trained and tested between the hours of 0700 and 1500.

Steroids Tested. The following substances obtained from the indicated suppliers were employed in the tests to be described: dehydroepiandrosterone (5-androsten-3β-ol-17-one), from Searle Chemical, Chicago, Illinois; estradiol (1,3,5(10)-estratrien-3,17β-diol), estrone (1,3,5(10)-estratrien-3-ol-17-one), testosterone (4-androsten-17β-ol-3-one), dihydrotestosterone (5α-androstan-17β-ol-3-one), androstenedione (4,androsten-3,17-dione), 17α-hydroxypregnenolone (5-pregnen-3β,17α-diol-20-one), and pregnenolone sulfate (5-pregnen-3β-ol-20-one sulfate, sodium salt) from Steraloids, Inc., Wilton, New Hampshire; aldosterone (4-pregnen-11β,21-diol-3,18,20-trione), pregnenolone (5-pregnen-3β-ol-20-one), progesterone (4-pregnen-3,20-dione) and DMSO (dimethylsulfoxide) from Sigma Chemical Company, St. Louis, Missouri. A gift of 16α-bromoepiandrosterone (5α-androstan-16α-bromo-3β-ol-17-one) was received from Dr. Paul Talalay, Johns Hopkins University School of Medicine, Baltimore, Maryland.

With the exception of pregnenolone sulfate, all of the substances were dissolved in appropriate amounts of pure DMSO and 2 µl of DMSO alone or containing test substance was injected icv into each mouse after training. Pregnenolone sulfate was dissolved in DMSO and diluted with physiological saline to a final concentration of 1% DMSO or less in saline, and the results of, injections of 2 µl of solutions containing pregnenolone sulfate were compared with those obtained with 2 µl of such saline solutions alone.

Apparatus, Training and Testing Procedures. The T-maze used for footshock active avoidance training (FAAT) consisted of a black plastic alley (46 cm long) with a start box at one end and two goal boxes (17.5 cm long) at the other. The start box was separated from the alley by a plastic guillotine door that prevented movement down the alley until training began. The alley was 12.5 cm deep and 9.8 cm wide. An electrifiable stainless steel rod floor ran throughout the maze.

Mice were not permitted to explore the maze before training. A block of training trials began when a mouse was placed in the start box. The guillotine door was raised and a muffled doorbell-type buzzer sounded simultaneously; footshock was 5 sec later through a scrambled grid floor shocker (Colbourn Instruments, model E13-08). The goal box first entered during the first set of trials was designated as "incorrect", and footshock was continued until the mouse entered the other goal box, which in all subsequent trials was designated "correct" for the particular mouse. At the end of each group of trials, the mouse was removed to its home cage.

As training proceeded, a mouse made one of two types of responses. A response latency longer than 5 sec was classed as an escape from the footshock. A response latency less than or equal to 5 sec was considered an avoidance, since the mouse avoided receiving a footshock. Two exclusion criteria were applied to reduce learning variability among mice, as follows. On the first training trials, mice with escape latencies greater than 20 sec were discarded. Mice not having at least one errorless escape latency between 1.5 and 3.5 sec on training trials 3 or 4 were excluded. The total exclusions were fewer than 15%. Mice received five such training trials. One week after training and post-trial administration of vehicle alone or vehicle containing test substance, T-maze training was resumed until each mouse made five avoidance responses in six consecutive training trials (trials to criterion). The recall score was taken to be the percentage of tested mice remembering original training.

Well-trained animals (recall score approximately 80%) were used to determine whether or not water-insoluble substances dissolves in DMSO could prevent amnesia induced by DMSO alone. In these instances, training was performed under conditions that tend to maximize learning (sound intensity, 65 decibels; footshock current, 0.35 mA; intertrial interval, 45 s). In the case of the water-soluble pregnenolone sulfate, for which it was desired to detect whether or not there was an enhancing effect on memory, training conditions were adjusted so that the initial recall score in vehicle controls (1% or less DMSO in saline) was only approximately 20% (sound intensity, 55 decibels; footshock current, 0.30 mA; intertrial interval, 30 s).

Surgical Procedure in Preparation for Intracerebroventricular (icv) Administration of Substances. Icv injection was the mode of administration of test substances because this eliminates problems of differential penetration of the blood-brain barrier. The following procedure was performed 24-48 hr prior to training. A single hole was drilled through the skull over the third ventricle (-0.5 mm relative to bregma, 0.5 mm right of central suture) while the mouse, appropriately anesthetized with methoxyflurane, was held in a stereotaxic instrument. The third ventricle was chosen as site of icv drug injection because only a single injection is required and the drug quickly reaches limbic system structures, believed to be associated with memorial processes. Immediately after training, mice were anesthetized with enflurane, a short acting anesthetic, and given an icv injection of 2 µl of vehicle alone or test substance in vehicle delivered over a 30-sec period through a 31-gauge needle attached to a 10-µl syringe; the injection was given within 2-3 min after the training. Accuracy of injection was determined to be greater than 95% by dye injection, monitored regularly.

Statistical Treatment of Data. All of the results are expressed in terms of the mean and standard errors of the mean (SEM). Significance of overall effects of treatment was determined by one-way analysis of variance (ANOVA) run on trials to criterion. Dunnett's t-test was used to make multiple comparisons of individual test groups with control groups. See Bruning, J.L., et al., In: Computational Handbook of Statistics, 2d ed., Scott, Foreman and Co., Glenview, pp. 18-30, 122-124, 128-130 (1977). Statistical comparison among experimental groups were made by Tukey's t-test. See Winer, B.J., In: Statistical Principles in Experimentation Design, 2d ed., McGraw-Hill, New York, pp. 196-210, 397-402 (1971).

### Results

Groups of 15 mice each were injected 2-3 min after FAAT with 3.5X10⁻¹⁰ mol of pregnenolone or with several other steroids indicated in Figures 1A and 1B or with vehicle alone (DMSO). Upon testing for retention of learning one week later, trials to criterion (mean ± SEM) for the DMSO-treated group (9.67 ± 0.33) were significantly greater than for the group receiving saline alone (6.80 ± 0.25; p < 0.01). Among the steroid-injected groups, those receiving pregnenolone, dehydroepiandrosterone (DHEA), dehydroepiandrosterone sulfate (DHEAS), androstenedione, testosterone, dihydrotestosterone, or aldosterone all showed significantly fewer trials to criterion than mice receiving DMSO alone (Figures 1A and 1B). The groups receiving progesterone, estrone, or estradiol gave results significantly different from those obtained with DMSO alone and significantly greater than those receiving saline alone (Figures 1A and 1B).

The experiments in Figures 1A and 1B were designed to test whether or not a particular steroid was a memory enhancer under our test conditions, but they were not suitable for testing the relative potencies of the substances. The dose employed, 3.5X10⁻¹⁰ mol, was based on our previous experiments with DHEA. See Roberts, Bologa, Flood and Smith, Brain Res. 406:357-362 (1987); Bologa, Sharma and Roberts, J.Neurosci.Res. 17:225-234 (1987); and Flood, Smith and Roberts, Brain Res. 447:269-278 (1988). Although DHEA was significantly active at lower doses, a higher effective dose was chosen so substances that might have weaker effects could be detected.

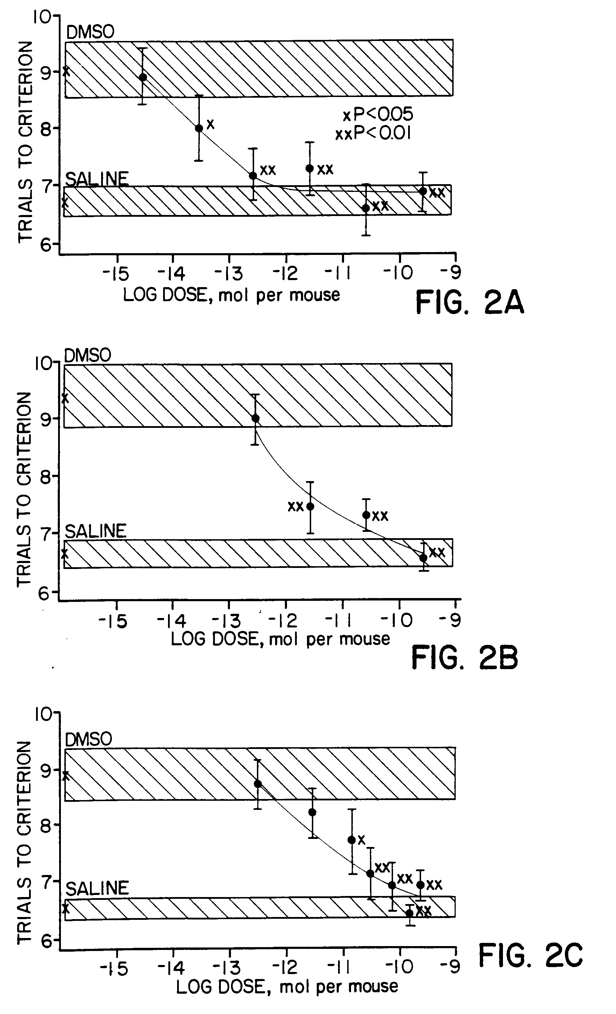

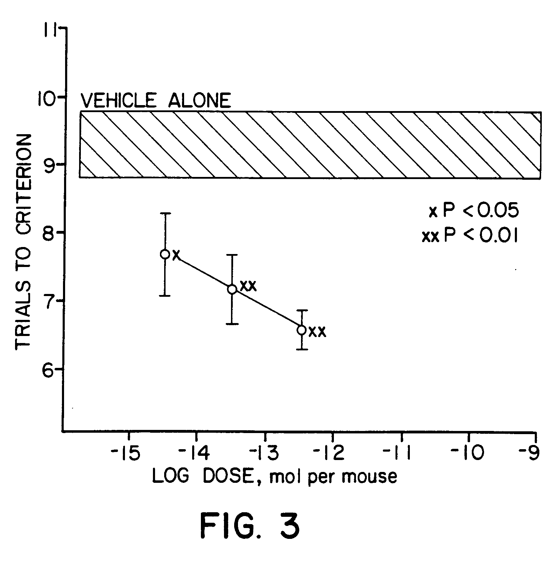

Dose-response curves were obtained from pregnenolone, DHEA, testosterone (Figures 2A-2C) and DHEAS (Figure 3) which are key substances in the metabolic sequence illustrated in Figures 1A and 1B. It will be noted that the doses used are plotted on a log scale. Pregnenolone was by far the most potent of the above three substances tested. A statistically significant memory enhancement was found at 3.5X10⁻¹⁴ mol per mouse (p < 0.05), and values virtually identical with the saline-injected animals were achieved at 3.5X10⁻¹³ mol per mouse and higher doses. Significant memory enhancement (p < 0.05 or less) was obtained with DHEA and testosterone at doses beginning at 3.5X10⁻¹² and 1.75X10⁻¹¹ mol per mouse, respectively. Thus, pregnenolone is at least 100 times more potent on a molar basis than the other two substances tested.

Weakly trained mice receiving a remarkably low does of pregnenolone, 3.5X10⁻¹⁵ mol per mouse, required significantly fewer trials to criterion (7.70±0.55; p < 0.05) than did the vehicle controls (9.30±0.47). Thus, pregnenolone sulfate is approximately 10 times more potent than pregnenolone and 1000 times more potent than DHEA.

The finding of the remarkable effects of pregnenolone and its sulfate in improving memory in mice shows that these substances exert specific cyberneticizing effects on the function of the brain, in which tissue both substances are normal constituents and where they are made from cholesterol and activity metabolized to other steroids. Results to date strongly suggest that pregnenolone and pregnenolone sulfate will be useful in treatment of neurological dysregulations caused by aging, autoimmune reactions, viral or bacterial infectious processes, or by physical injury.

Similarly, these substances will help in restoration and maintenance of optimal function of various tissues individually (e.g., muscle, bone, skin, cells of the immune and hematopoietic systems, etc.) and facilitate the optimization of their relations to each other. In this way, they will serve to improve general health, vigor, sexual activity and longevity.

Recently drugs have come into use that inhibit the synthesis of cholesterol so as to alleviate symptoms of arteriosclerosis. Some adverse reactions involving nervous system function and other body systems have begun to appear in individuals taking these drugs. Since cholesterol is a necessary precursor of pregnenolone, which in turn is the key precursor of steroid hormones, a block in cholesterol availability probably further embarrasses the already inadequate supply of steroid hormones in aging individuals. Pursuant to this invention, pregnenolone or pregnenolone sulfate is administered to individuals receiving drugs that block cholesterol synthesis, so that normal steroid biosynthesis may take place while arteriosclerotic processes are being ameliorated by these drugs.

Another aspect of this invention comprises a series of substances which has been designed to mimic the action of pregnenolone and pregnenolone sulfate on memory but which could not lead to the formation of other biologically active steroids. Such substances would have only memory-enhancing effects, and therefore would not intrude into the steroidal hormone economy of the organism, when this is not required.

### Dosage and Administration

Pursuant to this invention, pregnenolone or pregnenolone sulfate is administered orally, subcutaneously, intravenously, transcutaneously, intrathecally or intracisternally. For example, 10 to 50 mg, as capsules if administration is oral, may be administered to improve memory in individuals with benign memory deficit or with Alzheimer's disease. Like dosages may be administered to patients with memory deficits consequent from pathology. Pregnenolone or pregnenolone sulfate is also administered in accordance with the invention to individuals with multiple sclerosis to ameliorate the maladaptive attacks of the immune system on the nervous system, to patients with AIDS to improve their debilitated nervous system function, and to aging individuals to improve strength and enhance diminished sexual function.

## Claims

1. The use of pregnenolone or pregnenolone sulfate in the preparation of an agent to enhance the memory of a mammal.

2. The use as claimed in claim 1 to enhance the memory of a mammal having a memory deficit by correcting imbalance between the steroid hormones in said mammal.

3. The use as claimed in claim 1 or claim 2 in which said mammal is a mouse.

4. The use as claimed in claim 1 or claim 2 in which said mammal is a human.

5. The use as claimed in any one of claims 1 to 4 in which said pregnenolone or pregnenolone sulfate is adapted for oral, subcutaneous, intravenous, transcutaneous, intrathecal, intracerebroventricular or intracisternal administration.

## Patentansprüche

1. Verwendung von Pregnenolon oder Pregnenolonsulfat zur Herstellung eines Mittels zur Verbesserung des Gedächtnisses eines Säugetiers.

2. Verwendung nach Anspruch 1 zur Verbesserung des Gedächtnisses eines ein Gedächtnisdefizit aufweisenden Säugetiers durch Korrigieren des gestörten Gleichgewichts zwischen den Steroidhormonen dieses Säugetiers.

3. Verwendung nach Anspruch 1 oder 2, wobei dieses Säugetier eine Maus ist.

4. Verwendung nach Anspruch 1 oder 2, wobei dieses Säugetier ein Mensch ist.

5. Verwendung nach einem der Ansprüche 1 bis 4, wobei das Pregnenolon oder Pregnenolonsulfat zur oralen, subkutanen, intravenösen, perkutanen, intrathekalen, intracerebroventrikularen oder intracisternalen Verabreichung angepaßt ist.

## Revendications

1. Utilisation de pregnénolone ou de sulfate de pregnénolone pour préparer un agent destiné à renforcer la mémoire d'un mammifère.

2. Utilisation selon la revendication 1, pour renforcer la mémoire d'un mammifère ayant une insuffisance de mémoire, par correction du déséquilibre entre les hormones stéroïdiennes dudit mammifère.

3. Utilisation selon la revendication 1 ou 2, dans laquelle ledit mammifère est une souris.

4. Utilisation selon la revendication 1 ou 2, dans laquelle ledit mammifère est un être humain.

5. Utilisation selon l'une quelconque des revendications 1 à 4, dans laquelle ladite pregnénolone ou ledit sulfate de pregnénolone est adapté à une administration orale, sous-cutanée, intraveineuse, transcutanée, intrathécale, intracérébrovasculaire ou intracisternale.
